# EUROPEAN PATENT SPECIFICATION

(11) **EP 2 185 191 B1**
(45) Date of publication and mention of the grant of the patent: **12.09.2012**
(21) Application number: 08806940.6
(22) Date of filing: 27.06.2008
(51) Int. Cl.: A61K 39/145, A61P 31/16

(54) **LOW-ADDITIVE INFLUENZA VACCINES**
INFLUENZAIMPFSTOFFE MIT GERINGEM ZUSATZ
VACCINS ANTIGRIPPAUX À FAIBLE TENEUR EN ADDITIFS

(30) Priority: 27.06.2007 US 937515 P
(43) Date of publication of application: 19.05.2010
(60) Divisional application: 12166256.3
(73) Proprietor: Novartis AG, 4056 Basel (CH)
(72) Inventor: GREGERSEN, Jens-Peter, 35041 Marburg (DE); LUEBBEN, Holger, 35041 Marburg (DE); VORLOP, Juergen, 35041 Marburg (DE)
(74) Representative: Marshall, Cameron John
(86) International application number: PCT/IB2008/002238
(87) International publication number: WO 2009/001217

(56) References cited:
- EP-A- 1 605 052
- EP-A- 1 676 569
- WO-A-2007/052061
- WO-A-2007/052163
- DE-A1- 19 612 966
- US-A1- 2006 252 132
- REICHELDERFER P S ET AL: "REDUCTION OF ENDO TOXIN LEVELS IN INFLUENZA VIRUS VACCINES BY BARIUM SULFATE ADSORPTION ELUTION" APPLIED MICROBIOLOGY, vol. 30, no. 2, 1975, pages 333-334, XP002513140 ISSN: 0003-6919
- SANOFI PASTEUR: "Highlights of prescribing information Fluzone"[Online] 1980, pages 1-16, XP002513141 Retrieved from the Internet: URL:http://www.fda.gov/CBER/label/fluzoneL B.pdf> [retrieved on 2009-02-03]
- ID BIOMEDICAL CORPORATION OF QUEBEC: "Highlights of prescribing information Flulaval"[Online] 2006, pages 1-12, XP002513142 Retrieved from the Internet: URL:http://www.fda.gov/CBER/label/flulaval LB.pdf> [retrieved on 2009-02-03]
- MERTEN O-W ET AL: "PRODUCTION OF INFLUENZA VIRUS IN SERUM-FREE MAMMALIAN CELL CULTURES" DEVELOPMENTS IN BIOLOGICAL STANDARDIZATION, KARGER, BASEL, CH, vol. 98, 1 January 1997 (1997-01-01), pages 23-37, XP000909398 ISSN: 0301-5149
- KESSLER N ET AL: "SUITABILITY OF MDCK CELLS GROWN IN A SERUM-FREE MEDIUM FOR INFLUENZA VIRUS PRODUCTION" DEVELOPMENTS IN BIOLOGICAL STANDARDIZATION, KARGER, BASEL, CH, vol. 98, 1 January 1999 (1999-01-01), pages 13-21,73/74, XP001097586 ISSN: 0301-5149
- COX MANON M J ET AL: "Production of a novel influenza vaccine using insect cells: protection against drifted strains.", INFLUENZA AND OTHER RESPIRATORY VIRUSES JAN 2007 LNKD- PUBMED:19453478, vol. 1, no. 1, January 2007 (2007-01), pages 35-40, ISSN: 1750-2659
- NN: 'Optaflu Product details', [Online] 01 June 2007, EMEA Retrieved from the Internet: <URL:http://www.ema.europa.eu/ema/index.jsp ?curl=pages/medicines/human/medicines/00075 8/human_med_000952.jsp&murl=menus/medicines /medicines.jsp&mid=WC0b01ac058001d125>
- NN: 'Optaflu: authorisation documents - Scientific discussion', [Online] 01 June 2007, pages 1 - 31 EMEA Retrieved from the Internet: <URL:http://www.ema.europa.eu/docs/en_GB/do cument_library/EPAR_-_Scientific_Discussion /human/000758/WC500046954.pdf> [retrieved on 2011-12-23]
- KUSNICK C: "Zellkulturbasierter Influenzaimpfstoff ohne Hühnereiweiss /// Cell culture-based influenza vaccine without the white of hen's eggs", DEUTSCHE APOTHEKER ZEITUNG, DEUTSCHER APOTHEKER VERLAG, STUTTGART, DE, vol. 147, no. 25, 21 June 2007 (2007-06-21), pages 40-41, XP009155062, ISSN: 0011-9857

## Description

### TECHNICAL FIELD

This invention is in the field of vaccines for protecting against influenza virus infection, and in particular vaccines that contain low levels of pharmaceutical additives.

### BACKGROUND ART

Various forms of influenza virus vaccine are currently available (*e.g*. see chapters 17 & 18 of reference 1). Vaccines are generally based either on live virus or on inactivated virus. Inactivated vaccines may be based on whole virions, 'split' virions, or on purified surface antigens.

In addition to their antigenic content, current influenza vaccines include various pharmaceutical additives and other contaminants, such as: anti-bacterial preservatives *e.g*. thimerosal; detergents *e.g*. CTAB, polysorbate 80, octoxynol 10, *etc*.; antibiotics *e.g*. neomycin, kanamycin; formaldehyde; and egg-derived materials including egg proteins (*e.g*. ovomucoid) and chicken DNA.

For the 2006/07 season, for instance, manufacturers' datasheets for the four inactivated vaccines used in the USA reveal the following information:

| **Vaccine** | **Preservative** | **Antibiotic(s)** | **Formaldehyde** | **Egg materials** |
|---|---|---|---|---|
| Fluarix^{™} | Thimerosal | Gentamycin | Yes | Yes |
| Fluvirin^{™} | Thimerosal | No | No | Yes |
| Fluzone^{™} | Optional thimerosal | No | Yes | Yes |
| FluLaval^{™} | Thimerosal | No | Yes | Yes |

Similar to the Fluvirin^{™} 2006-07 product, reference 2 prepared a vaccine that was free of formaldehyde, but contained thimerosal and egg products. Reference 59 uses BPL, rather than formaldehyde, to inactivate influenza viruses grown in cell culture. The vaccine can be free from mercury.

It is an object of the invention to provide further and improved influenza vaccines, and processes for their manufacture, which reduce or eliminate the amount and/or number of these pharmaceutical additives, thereby giving a purer vaccine product.

### DISCLOSURE OF THE INVENTION

According to the invention, an influenza vaccine lacks a mercurial preservative; an antibiotic; formaldehyde; and egg-derived materials.

Thus in one embodiment the invention provides a sterile vaccine comprising an inactivated influenza virus antigen, wherein the vaccine contains no mercurial preservative, no antibiotic no formaldehyde and no egg-derived materials.

Preferred vaccines also have a very low endotoxin content *e.g*. less than 0.1 IU/ml, and preferably less than 0.05 IU/ml. The international unit for endotoxin measurement is well known and can be calculated for a sample by, for instance, comparison to an international standard [3,4], such as the *2nd International Standard* (Code 94/580 - IS) available from the NIBSC. Current vaccines prepared from virus grown in eggs have endotoxin levels in the region of 0.5-5 IU/ml.

The invention also provides a process for preparing a sterile influenza virus antigen formulation, comprising the steps of: (i) growing influenza virus in a cell culture system, in the absence of egg-derived materials and of antibiotics; (ii) inactivating the influenza viruses grown in step (i), in the absence of formaldehyde; and (iii) preparing a vaccine antigen formulation from the inactivated influenza viruses, in the absence of thimerosal. The resulting antigen formulation may a bulk vaccine antigen that can be used to prepare monovalent or multivalent vaccines.

### Antigen components

The invention uses influenza virus antigens prepared from influenza virions.

The virions are inactivated without using formaldehyde. Chemical means for inactivating a virus include treatment with an effective amount of one or more of the following agents: detergents, β-propiolactone, or UV light. Additional chemical means for inactivation include treatment with methylene blue, psoralen, carboxyfullerene (C60) or a combination of any thereof. Other methods of viral inactivation are known in the art, such as for example binary ethylamine, acetyl ethyleneimine, or gamma irradiation.

Virions can be harvested from virus-containing fluids by various methods. For example, a purification process may involve zonal centrifugation using a linear sucrose gradient solution that includes detergent to disrupt the virions. Antigens may then be purified, after optional dilution, by diafiltration.

Split virions are obtained by treating purified virions with detergents (*e.g*. ethyl ether, polysorbate 80, deoxycholate, tri-*N*-butyl phosphate, Triton X-100, Triton N101, cetyltrimethylammonium bromide, Tergitol NP9, *etc*.) to produce subvirion preparations, including the 'Tween-ether' splitting process. Methods of splitting influenza viruses are well known in the art *e.g*. see refs. 5-10, *etc.* Splitting of the virus is typically carried out by disrupting or fragmenting whole virus, whether infectious or non-infectious, with a disrupting concentration of a splitting agent. The disruption results in a full or partial solubilisation of the virus proteins, altering the integrity of the virus. Preferred splitting agents are non-ionic and ionic (*e.g*. cationic) surfactants *e.g*. alkylglycosides, alkylthioglycosides, acyl sugars, sulphobetaines, betains, polyoxyethylenealkylethers, N,N-dialkyl-Glucamides, Hecameg, alkylphenoxy-polyethoxyethanols, quaternary ammonium compounds, sarcosyl, CTABs (cetyl trimethyl ammonium bromides), tri-*N-*butyl phosphate, Cetavlon, myristyltrimethylammonium salts, lipofectin, lipofectamine, and DOTMA, the octyl- or nonylphenoxy polyoxyethanols (*e.g*. the Triton surfactants, such as Triton X-100 or Triton N101), polyoxyethylene sorbitan esters (the Tween surfactants), polyoxyethylene ethers, polyoxyethlene esters, *etc.* One useful splitting procedure uses the consecutive effects of sodium deoxycholate and formaldehyde, and splitting can take place during initial virion purification (*e.g*. in a sucrose density gradient solution). Split virions can usefully be resuspended in sodium phosphate-buffered isotonic sodium chloride solution.

Purified surface antigen vaccines comprise the influenza surface antigens haemagglutinin and, typically, also neuraminidase. Processes for preparing these proteins in purified form are well known.

Influenza antigens can also be presented in the form of virosomes [11] (nucleic acid free viral-like liposomal particles), as in the INFLEXAL V™ and INVAVAC™ products.

The influenza virus may be attenuated. The influenza virus may be temperature-sensitive. The influenza virus may be cold-adapted. These three features are, however, associated more with live virus vaccines.

Human influenza virus strains for use in vaccines change from season to season. In the current inter-pandemic period, vaccines typically include two influenza A strains (H1N1 and H3N2) and one influenza B strain, and trivalent vaccines are typical. The invention may also use HA from pandemic strains (*i.e*. strains to which the vaccine recipient and the general human population are immunologically naïve), such as H2, H5, H7 or H9 subtype strains (in particular of influenza A virus), and influenza vaccines for pandemic strains may be monovalent or may be based on a normal trivalent vaccine supplemented by a pandemic strain. Depending on the season and on the nature of the antigen included in the vaccine, however, the invention may protect against one or more of HA subtypes H1, H2, H3, H4, H5, H6, H7, H8, H9, H10, H11, H12, H13, H14, H15 or H16 (influenza A virus). The invention may protect against one or more of influenza A virus NA subtypes N1, N2, N3, N4, N5, N6, N7, N8 or N9.

As well as being suitable for immunizing against inter-pandemic strains, the compositions of the invention are particularly useful for immunizing against pandemic strains. The characteristics of an influenza strain that give it the potential to cause a pandemic outbreak are: (a) it contains a new hemagglutinin compared to the hemagglutinins in currently circulating human strains *i.e*. one that either has not been evident in the human population for over a decade (*e.g*. H2) or has not previously been seen at all in the human population (*e.g*. H5, H6 or H9, that have generally been found only in bird populations), and/or it contains a new neuraminidase compared to the neuraminidases in currently-circulating human strains, such that the human population will be immunologically naïve to the strain's hemagglutinin and/or neuraminidase; (b) it is capable of being transmitted horizontally in the human population; and (c) it is pathogenic to humans. A virus with H5 haemagglutinin type is preferred for immunizing against pandemic influenza, such as a H5N1 strain. Other possible strains include H5N3, H9N2, H2N2, H7N1 and H7N7, and any other emerging potentially pandemic strains.

Within the H5 subtype, a virus may fall into HA clade 1, HA clade 1', HA clade 2 or HA clade 3 [12], with clades 1 and 3 being particularly relevant.

Other strains whose antigens can usefully be included in the compositions are strains which are resistant to antiviral therapy (*e.g*. resistant to oseltamivir [13] and/or zanamivir), including resistant pandemic strains [14].

Compositions of the invention may include antigen(s) from one or more (*e.g*. 1, 2, 3, 4 or more) influenza virus strains, including influenza A virus and/or influenza B virus. Monovalent vaccines can be prepared, as can 2-valent, 3-valent, 4-valent, *etc*.. Where a vaccine includes more than one strain of influenza, the different strains are typically grown separately and are mixed after the viruses have been harvested and antigens have been prepared. Thus a process of the invention may include the step of mixing antigens from more than one influenza strain, and this process may be performed under non-refrigerated conditions. A trivalent vaccine is preferred, including antigens from two influenza A virus strains and one influenza B virus strain.

In some embodiments of the invention, the compositions may include antigen from a single influenza A strain. In some embodiments, the compositions may include antigen from two influenza A strains, provided that these two strains are not H1N1 and H3N2. In some embodiments, the compositions may include antigen from more than two influenza A strains.

The influenza virus may be a reassortant strain, and may have been obtained by reverse genetics techniques. Reverse genetics techniques [*e.g*. 15-19] allow influenza viruses with desired genome segments to be prepared *in vitro* using plasmids. Typically, it involves expressing (a) DNA molecules that encode desired viral RNA molecules *e.g*. from polI promoters, and (b) DNA molecules that encode viral proteins *e.g*. from polII promoters, such that expression of both types of DNA in a cell leads to assembly of a complete intact infectious virion. The DNA preferably provides all of the viral RNA and proteins, but it is also possible to use a helper virus to provide some of the RNA and proteins. Plasmid-based methods using separate plasmids for producing each viral RNA are preferred [20-22], and these methods will also involve the use of plasmids to express all or some (*e.g*. just the FB1, PB2, PA and NP proteins) of the viral proteins, with up to 12 plasmids being used in some methods. To reduce the number of plasmids needed, a recent approach [23] combines a plurality of RNA polymerase 1 transcription cassettes (for viral RNA synthesis) on the same plasmid (*e.g*. sequences encoding 1, 2, 3, 4, 5, 6, 7 or all 8 influenza A vRNA segments), and a plurality of protein-coding regions with RNA polymerase II promoters on another plasmid (*e.g*. sequences encoding 1, 2, 3, 4, 5, 6, 7 or all 8 influenza A mRNA transcripts). Preferred aspects of the reference 23 method involve: (a) PB1, PB2 and PA mRNA-encoding regions on a single plasmid; and (b) all 8 vRNA-encoding segments on a single plasmid. Including the NA and HA segments on one plasmid and the six other segments on another plasmid can also facilitate matters.

As an alternative to using poll promoters to encode the viral RNA segments, it is possible to use bacteriophage polymerase promoters [24]. For instance, promoters for the SP6, T3 or T7 polymerases can conveniently be used. Because of the species-specificity of polI promoters, bacteriophage polymerase promoters can be more convenient for many cell types (*e.g*. MDCK), although a cell must also be transfected with a plasmid encoding the exogenous polymerase enzyme.

In other techniques it is possible to use dual polI and polII promoters to simultaneously code for the viral RNAs and for expressible mRNAs from a single template [25,26].

Thus the virus, particularly an influenza A virus, may include one or more RNA segments from a A/PR/8/34 virus (typically 6 segments from A/PR/8/34, with the HA and N segments being from a vaccine strain, *i.e*. a 6:2 reassortant). It may also include one or more RNA segments from a A/WSN/33 virus, or from any other virus strain useful for generating reassortant viruses for vaccine preparation. Typically, the invention protects against a strain that is capable of human-to-human transmission, and so the strain's genome will usually include at least one RNA segment that originated in a mammalian (*e.g*. in a human) influenza virus. It may include NS segment that originated in an avian influenza virus.

As mentioned above, the viruses used as the source of the antigens are generally grown on cell culture, thereby avoiding contamination with components from embryonated hen eggs. Thus vaccines of the invention can be free from chicken DNA, as well as being free from egg proteins (such as ovalbumin and ovomucoid).

The cell substrate will typically be a cell line of mammalian origin. Suitable mammalian cells of origin include, but are not limited to, hamster, cattle, primate (including humans and monkeys) and dog cells. Various cell types may be used, such as kidney cells, fibroblasts, retinal cells, lung cells, *etc.* Examples of suitable hamster cells are the cell lines having the names BHK21 or HKCC. Suitable monkey cells are *e.g*. African green monkey cells, such as kidney cells as in the Vero cell line. Suitable dog cells are *e.g*. kidney cells, as in the MDCK cell line. Thus suitable cell lines include, but are not limited to: MDCK; CHO; 293T; BHK; Vero; MRC-5; PER.C6; WI-38; *etc.*

Preferred mammalian cell lines for growing influenza viruses include: MDCK cells [27-30], derived from Madin Darby canine kidney; Vero cells [31-33], derived from African green monkey *(Cercopithecus aethiops)* kidney; or PER.C6 cells [34], derived from human embryonic retinoblasts. These cell lines are widely available *e.g*. from the American Type Cell Culture (ATCC) collection [35], from the Coriell Cell Repositories [36], or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940. As an alternative to mammalian cell lines, virus can be grown on avian cell lines [*e.g.* refs. 37-39], including avian embryonic stem cells [37,40] and cell lines derived from ducks (*e.g*. duck retina), or from hens. Suitable avian embryonic stem cells, include the EBx cell line derived from chicken embryonic stem cells, EB45, EB14, and EB14-074 [41]. Chicken embryo fibroblasts (CEF), can also be used, *etc.*

The most preferred cell lines for growing influenza viruses are MDCK cell lines. The original MDCK cell line is available from the ATCC as CCL-34, but derivatives of this cell line may also be used. For instance, reference 27 discloses a MDCK cell line that was adapted for growth in suspension culture ('MDCK 33016', deposited as DSM ACC 2219). Similarly, reference 42 discloses a MDCK-derived cell line that grows in suspension in serum-free culture ('B-702', deposited as FERM BP-7449). Reference 43 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (PTA-6503). Reference 44 discloses MDCK cell lines with high susceptibility to infection, including 'MDCK.5F1' cells (ATCC CRL-12042). Any of these MDCK cell lines can be used.

The culture for cell growth, and also the viral inoculum used to start the culture, will preferably be free from (*i.e.* will have been tested for and given a negative result for contamination by) herpes simplex virus, respiratory syncytial virus, parainfluenza virus 3, SARS coronavirus, adenovirus, rhinovirus, reoviruses, polyomaviruses, birnaviruses, circoviruses, and/or parvoviruses [45]. Absence of herpes simplex viruses is particularly preferred.

Virus may be grown on cells in suspension [27,46,47] or in adherent culture. In one embodiment, the cells may be adapted for growth in suspension. One suitable MDCK cell line that is adapted for growth in suspension culture is MDCK 33016 (deposited as DSM ACC 2219). As an alternative, microcarrier culture can be used.

Cell lines supporting influenza virus replication are preferably grown in serum-free culture media and/or protein free media. A medium is referred to as a serum-free medium in the context of the present invention in which there are no additives from serum of human or animal origin. Protein-free is understood to mean cultures in which multiplication of the cells occurs with exclusion of proteins, growth factors, other protein additives and non-serum proteins, but can optionally include proteins such as trypsin or other proteases that may be necessary for viral growth. The cells growing in such cultures naturally contain proteins themselves.

Cell lines supporting influenza virus replication are preferably grown below 37°C [48] (*e.g*. 30-36°C) during viral replication.

The method for propagating virus in cultured cells generally includes the steps of inoculating the cultured cells with the strain to be cultured, cultivating the infected cells for a desired time period for virus propagation, such as for example as detennined by virus titer or antigen expression (*e.g*. between 24 and 168 hours after inoculation) and collecting the propagated virus. The cultured cells are inoculated with a virus (measured by PFU or TCID₅₀) to cell ratio of 1:500 to 1:1, preferably 1:100 to 1:5, more preferably 1:50 to 1:10. The virus is added to a suspension of the cells or is applied to a monolayer of the cells, and the virus is absorbed on the cells for at least 60 minutes but usually less than 300 minutes, preferably between 90 and 240 minutes at 25°C to 40°C, preferably 28°C to 37°C. The infected cell culture (*e.g*. monolayers) may be removed either by freeze-thawing or by enzymatic action to increase the viral content of the harvested culture supernatants. The harvested fluids are then either inactivated or stored frozen. Cultured cells may be infected at a multiplicity of infection ("m.o.i.") of about 0.0001 to 10, preferably 0.002 to 5, more preferably to 0.001 to 2. Still more preferably, the cells are infected at a m.o.i of about 0.01. Infected cells may be harvested 30 to 60 hours post infection. Preferably, the cells are harvested 34 to 48 hours post infection. Still more preferably, the cells are harvested 38 to 40 hours post infection. Proteases (typically trypsin) are generally added during cell culture to allow viral release, and the proteases can be added at any suitable stage during the culture. According to the invention, antibiotics can be avoided during the culture.

Influenza vaccines are currently standardised by reference to HA levels, typically measured by SRID. Existing vaccines typically contain about 15µg of HA per strain, although lower doses can be used (*e.g*. when using an adjuvant). Fractional doses such as ½ (*i.e.* 7.5 µg HA per strain), ¼ and ¹/₈ have been used [62,63], as have higher doses (*e.g*. 3x or 9x doses [49,50]). Thus vaccines may include between 0.1 and 150µg of HA per influenza strain, preferably between 0.1 and 50µg *e.g.* 0.1-20µg, 0.1-15µg, 0.1-10µg, 0.1-7.5µg, 0.5-5µg, *etc*. Particular doses include *e.g*. about 90, about 45, about 30, about 15, about 10, about 7.5, about 5, about 3.8, about 1.9, about 1.5, *etc.* per strain. The components of the vaccines, kits and processes of the invention (*e.g*. their volumes and concentrations) may be selected to provide these antigen doses in final products.

HA used with the invention may be a natural HA as found in a virus, or may have been modified. For instance, it is known to modify HA to remove determinants (*e.g*. hyper-basic regions, such as around the cleavage site between HA1 and HA2).

As well as including haemagglutinin, compositions of the invention may include further influenza virus proteins. For instance, they will typically include neuraminidase glycoprotein. They may also include a matrix protein, such as M1 and/or M2 (or a fragment thereof), and/or nucleoprotein.

### Host cell DNA

Where virus has been grown on a cell line then it is standard practice to minimize the amount of residual cell line DNA in the final vaccine, in order to minimize any oncogenic activity of the DNA. Thus the composition preferably contains less than 10ng (preferably less than 1ng, and more preferably less than 100pg) of residual host cell DNA per dose, although trace amounts of host cell DNA may be present. In general, the host cell DNA that it is desirable to exclude from compositions of the invention is DNA that is longer than 100bp.

Measurement of residual host cell DNA is now a routine regulatory requirement for biologicals and is within the normal capabilities of the skilled person. The assay used to measure DNA will typically be a validated assay [51,52]. The performance characteristics of a validated assay can be described in mathematical and quantifiable terms, and its possible sources of error will have been identified. The assay will generally have been tested for characteristics such as accuracy, precision, specificity. Once an assay has been calibrated (*e.g*. against known standard quantities of host cell DNA) and tested then quantitative DNA measurements can be routinely performed. Three principle techniques for DNA quantification can be used: hybridization methods, such as Southern blots or slot blots [53]; immunoassay methods, such as the Threshold™ System [54]; and quantitative PCR [55]. These methods are all familiar to the skilled person, although the precise characteristics of each method may depend on the host cell in question *e.g*. the choice of probes for hybridization, the choice of primers and/or probes for amplification, *etc.* The Threshold™ system from *Molecular Devices* is a quantitative assay for picogram levels of total DNA, and has been used for monitoring levels of contaminating DNA in biopharmaceuticals [54]. A typical assay involves non-sequence-specific formation of a reaction complex between a biotinylated ssDNA binding protein, a urease-conjugated anti-ssDNA antibody, and DNA. All assay components are included in the complete Total DNA Assay Kit available from the manufacturer. Various commercial manufacturers offer quantitative PCR assays for detecting residual host cell DNA *e.g*. AppTec™ Laboratory Services, BioReliance™, Althea Technologies, *etc.* A comparison of a chemiluminescent hybridisation assay and the total DNA Threshold™ system for measuring host cell DNA contamination of a human viral vaccine can be found in reference 56.

Contaminating DNA can be removed during vaccine preparation using standard purification procedures *e.g.* chromatography, *etc.* Removal of residual host cell DNA can be enhanced by nuclease treatment *e.g*. by using a DNase. A convenient method for reducing host cell DNA contamination is disclosed in references 57 & 58, involving a two-step treatment, first using a DNase (*e.g*. Benzonase), which may be used during viral growth, and then a cationic detergent (*e.g*. CTAB), which may be used during virion disruption. Treatment with an alkylating agent, such as β-propiolactone, can also be used to remove host cell DNA, and advantageously may also be used to inactivate virions [59] while avoiding use of formaldehyde.

Vaccines containing <10ng (*e.g.* <1ng, <100pg) host cell DNA per 15µg of haemagglutinin are preferred, as are vaccines containing <10ng (*e.g*. <1ng, <100pg) host cell DNA per 0.25ml volume. Vaccines containing <10ng (*e.g*. <1ng, <100pg) host cell DNA per 50µg of haemagglutinin are more preferred, as are vaccines containing <10ng (*e.g*. <1ng, <100pg) host cell DNA per 0.5ml volume.

### Adjuvants

Compositions of the invention may advantageously include an adjuvant, which can function to enhance the immune responses (humoral and/or cellular) elicited in a patient who receives the composition. The use of adjuvants with influenza vaccines has been described before. In references 60 & 61, aluminum hydroxide was used, and in reference 62, a mixture of aluminum hydroxide and aluminum phosphate was used. Reference 63 also described the use of aluminum salt adjuvants. The FLUAD™ product from Chiron Vaccines includes an oil-in-water emulsion.

Adjuvants that can be used with the invention include, but are not limited to:
- A mineral-containing composition, including calcium salts and aluminum salts (or mixtures thereof). Calcium salts include calcium phosphate (*e.g*. the "CAP" particles disclosed in ref. 64). Aluminum salts include hydroxides, phosphates, sulfates, *etc.,* with the salts taking any suitable form (*e.g*. gel, crystalline, amorphous, *etc*.). Adsorption to these salts is preferred. The mineral containing compositions may also be formulated as a particle of metal salt [65]. Aluminum salt adjuvants are described in more detail below.
- Cytokine-inducing agents (see in more detail below).
- Saponins [chapter 22 of ref. 101], which are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™. Saponin compositions have been purified using HPLC and RP-HPLC. Specific purified fractions using these techniques have been identified, including QS7, QS 17, QS18, QS21, QH-A, QH-B and QH-C. Preferably, the saponin is QS21. A method of production of QS21 is disclosed in ref. 66. It is possible to use fraction A of Quil A together with at least one other adjuvant [67]. Saponin formulations may also comprise a sterol, such as cholesterol [68]. Combinations of saponins and cholesterols can be used to form unique particles called immunostimulating complexs (ISCOMs) [chapter 23 of ref. 101]. ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in refs. 68-70. Optionally, the ISCOMS may be devoid of additional detergent [71]. It is possible to use a mixture of at least two ISCOM complexes, each complex comprising essentially one saponin fraction, where the complexes are ISCOM complexes or ISCOM matrix complexes [72]. A review of the development of saponin based adjuvants can be found in refs. 73 & 74.
- Fatty adjuvants (see in more detail below).
- Bacterial ADP-ribosylating toxins (*e.g*. the *E.coli* heat labile enterotoxin "LT", cholera toxin "CT", or pertussis toxin "PT") and detoxified derivatives thereof, such as the mutant toxins known as LT-K63 and LT-R72 [75]. The use of detoxified ADP-ribosylating toxins as mucosal adjuvants is described in ref. 76 and as parenteral adjuvants in ref. 77.
- Bioadhesives and mucoadhesives, such as esterified hyaluronic acid microspheres [78] or chitosan and its derivatives [79].
- Microparticles (*i.e*. a particle of ~100nm to ~150µm in diameter, more preferably ~200nm to ~30µm in diameter, or ~500nm to ~10µm in diameter) formed from materials that are biodegradable and non-toxic (*e.g*. a poly(α-hydroxy acid), a polyhydroxybutyric acid, a polyorthoester, a polyanhydride, a polycaprolactone, *etc*.), with poly(lactide-co-glycolide) being preferred, optionally treated to have a negatively-charged surface (*e.g*. with SDS) or a positively-charged surface (*e.g*. with a cationic detergent, such as CTAB).
- Liposomes (Chapters 13 & 14 of ref. 101). Examples of liposome formulations suitable for use as adjuvants are described in refs. 80-82.
- Oil-in-water emulsions (see in more detail below).
- Polyoxyethylene ethers and polyoxyethylene esters [83]. Such formulations further include polyoxyethylene sorbitan ester surfactants in combination with an octoxynol [84] as well as polyoxyethylene alkyl ethers or ester surfactants in combination with at least one additional non-ionic surfactant such as an octoxynol [85]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.
- Muramyl peptides, such as N-acetylmuramyl-L-threonyl-D-isoglutamine ("thr-MDP"), N-acetyl-normuramyl-L-alanyl-D-isoglutamine (nor-MDP), N-acetylglucsaminyl-N-acetylmuramyl-L-Al-D-isoglu-L-Ala-dipalmitoxy propylamide ("DTP-DPP", or "Theramide™), N-acetylmuramyl-L-alanyl-D-isoglutaminyl-L-alanine-2-(1'-2'dipalmitoyl-sn-glycero-3-hydroxyphosphoryloxy)-ethylamine ("MTP-PE").
- An outer membrane protein proteosome preparation prepared from a first Gram-negative bacterium in combination with a liposaccharide preparation derived from a second Gram-negative bacterium, wherein the outer membrane protein proteosome and liposaccharide preparations form a stable non-covalent adjuvant complex. Such complexes include "IVX-908", a complex comprised of *Neisseria meningitidis* outer membrane and lipopolysaccharides. They have been used as adjuvants for influenza vaccines [86].
- Methyl inosine 5'-monophosphate ("MIMP") [87].
- A polyhydroxlated pyrrolizidine compound [88], such as one having formula: 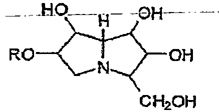 where R is selected from the group comprising hydrogen, straight or branched, unsubstituted or substituted, saturated or unsaturated acyl, alkyl (*e.g*. cycloalkyl), alkenyl, alkynyl and aryl groups, or a pharmaceutically acceptable salt or derivative thereof. Examples include, but are not limited to: casuarine, casuarine-6-α-D-glucopyranose, 3-epi-casuarine, 7-epi-casuarine, 3,7-diepi-casuarine, *etc*.
- A gamma inulin [89] or derivative thereof, such as algammulin.
- A CD1d ligand, such as a α-glycosylceramide *e.g*. α-galactosylceramide.

These and other adjuvant-active substances are discussed in more detail in references 101 & 102.

Compositions may include two or more of said adjuvants. For example, they may advantageously include both an oil-in-water emulsion and a cytokine-inducing agent, as this combination improves the cytokine responses elicited by influenza vaccines, such as the interferon-γ response, with the improvement being much greater than seen when either the emulsion or the agent is used on its own.

Antigens and adjuvants in a composition will typically be in admixture.

Where a vaccine includes an adjuvant, it may be prepared extemporaneously, at the time of delivery. Thus the invention provides kits including the antigen and adjuvant components ready for mixing. The kit allows the adjuvant and the antigen to be kept separately until the time of use. The components are physically separate from each other within the kit, and this separation can be achieved in various ways. For instance, the two components may be in two separate containers, such as vials. The contents of the two vials can then be mixed *e.g*. by removing the contents of one vial and adding them to the other vial, or by separately removing the contents of both vials and mixing them in a third container. In a preferred arrangement, one of the kit components is in a syringe and the other is in a container such as a vial. The syringe can be used (*e.g*. with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration. In another preferred arrangement, the two kit components are held together but separately in the same syringe *e.g*. a dual-chamber syringe, such as those disclosed in references 90-97 *etc.* When the syringe is actuated (*e.g*. during administration to a patient) then the contents of the two chambers are mixed. This arrangement avoids the need for a separate mixing step at the time of use.

### Oil-in-water emulsion adjuvants

Oil-in-water emulsions have been found to be particularly suitable for use in adjuvanting influenza virus vaccines. Various such emulsions are known, and they typically include at least one oil and at least one surfactant, with the oil(s) and surfactant(s) being biodegradable (metabolisable) and biocompatible. The oil droplets in the emulsion are generally less than 5µm in diameter, and may even have a sub-micron diameter, with these small sizes being achieved with a microfluidiser to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e.g*. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Preferred surfactants for including in the emulsion are Tween 80 (polyoxyethylene sorbitan monooleate), Span 85 (sorbitan trioleate), lecithin and Triton X-100. As mentioned above, detergents such as Tween 80 may contribute to the thermal stability seen in the examples below.

Mixtures of surfactants can be used *e.g*. Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Specific oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [98-100], as described in more detail in Chapter 10 of ref. 101 and chapter 12 of ref. 102. The MF59 emulsion advantageously includes citrate ions *e.g*. 10mM sodium citrate buffer.
- An emulsion of squalene, a tocopherol, and Tween 80. The emulsion may include phosphate buffered saline. It may also include Span 85 (*e.g*. at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. Squalene and Tween 80 may be present volume ratio of about 5:2. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g*. with an average diameter of between 100 and 250nm, preferably about 180nm.
- An emulsion of squalene, a tocopherol, and a Triton detergent (*e.g*. Triton X-100). The emulsion may also include a 3d-MPL (see below). The emulsion may contain a phosphate buffer.
- An emulsion comprising a polysorbate (*e.g*. polysorbate 80), a Triton detergent (*e.g*. Triton X-100) and a tocopherol (*e.g*. an α-tocopherol succinate). The emulsion may include these three components at a mass ratio of about 75:11:10 (*e.g*. 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [103] (0.05-1% Thr-MDP, 5% squalane, 2.5% Pluronic L121 and 0.2% polysorbate 80). It can also be used without the Thr-MDP, as in the "AF" adjuvant [104] (5% squalane, 1.25% Pluronic L121 and 0.2% polysorbate 80). Microfluidisation is preferred.
- An emulsion comprising squalene, an aqueous solvent, a polyoxyethylene alkyl ether hydrophilic nonionic surfactant (*e.g*. polyoxyethylene (12) cetostearyl ether) and a hydrophobic nonionic surfactant (*e.g*. a sorbitan ester or mannide ester, such as sorbitan monoleate or 'Span 80'). The emulsion is preferably thermoreversible and/or has at least 90% of the oil droplets (by volume) with a size less than 200 nm [105]. The emulsion may also include one or more of: alditol; a cryoprotective agent (*e.g*. a sugar, such as dodecylmaltoside and/or sucrose); and/or an alkylpolyglycoside. Such emulsions may be lyophilized.
- An emulsion having from 0.5-50% of an oil, 0.1-10% of a phospholipid, and 0.05-5% of a non-ionic surfactant. As described in reference 106, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 107, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion comprising a mineral oil, a non-ionic lipophilic ethoxylated fatty alcohol, and a non-ionic hydrophilic surfactant (*e.g*. an ethoxylated fatty alcohol and/or polyoxyethylene-polyoxypropylene block copolymer) [108].
- An emulsion comprising a mineral oil, a non-ionic hydrophilic ethoxylated fatty alcohol, and a non-ionic lipophilic surfactant (*e.g*. an ethoxylated fatty alcohol and/or polyoxyethylene-polyoxypropylene block copolymer) [108].
- An emulsion in which a saponin (*e.g*. QuilA or QS21) and a sterol (*e.g.* a cholesterol) are associated as helical micelles [109].

The emulsions may be mixed with antigen extemporaneously, at the time of delivery. Thus the adjuvant and antigen may be kept separately in a packaged or distributed vaccine, ready for final formulation at the time of use. The antigen will generally be in an aqueous form, such that the vaccine is finally prepared by mixing two liquids. The volume ratio of the two liquids for mixing can vary (*e.g*. between 5:1 and 1:5) but is generally about 1:1.

After the antigen and adjuvant have been mixed, haemagglutinin antigen will generally remain in aqueous solution but may distribute itself around the oil/water interface. In general, little if any haemagglutinin will enter the oil phase of the emulsion.

Where a composition includes a tocopherol, any of the α, β, γ, δ, ε or ξ tocopherols can be used, but α-tocopherols are preferred. The tocopherol can take several forms *e.g*. different salts and/or isomers. Salts include organic salts, such as succinate, acetate, nicotinate, *etc*. D-α-tocopherol and DL-α-tocopherol can both be used. Tocopherols are advantageously included in vaccines for use in elderly patients (*e.g*. aged 60 years or older) because vitamin E has been reported to have a positive effect on the immune response in this patient group [110]. They also have antioxidant properties that may help to stabilize the emulsions [111]. A preferred α-tocopherol is DL-α-tocopherol, and the preferred salt of this tocopherol is the succinate. The succinate salt has been found to cooperate with TNF-related ligands *in vivo.* Moreover, α-tocopherol succinate is known to be compatible with influenza vaccines and to be a useful preservative as an alternative to mercurial compounds [8].

### Cytokine-inducing agents

Cytokine-inducing agents for inclusion in compositions of the invention are able, when administered to a patient, to elicit the immune system to release cytokines, including interferons and interleukins. Cytokine responses are known to be involved in the early and decisive stages of host defense against influenza infection [112]. Preferred agents can elicit the release of one or more of: interferon-γ; interleukin-1; interleukin-2; interleukin-12; TNF-α; TNF-β; and GM-CSF. Preferred agents elicit the release of cytokines associated with a Thl-type immune response *e.g*. interferon-γ, TNF-α, interleukin-2. Stimulation of both interferon-γ and interleukin-2 is preferred.

As a result of receiving a composition of the invention, therefore, a patient will have T cells that, when stimulated with an influenza antigen, will release the desired cytokine(s) in an antigen-specific manner. For example, T cells purified form their blood will release γ-interferon when exposed *in vitro* to influenza virus haemagglutinin. Methods for measuring such responses in peripheral blood mononuclear cells (PBMC) are known in the art, and include ELISA, ELISPOT, flow-cytometry and real-time PCR. For example, reference 113 reports a study in which antigen-specific T cell-mediated immune responses against tetanus toxoid, specifically γ-interferon responses, were monitored, and found that ELISPOT was the most sensitive method to discriminate antigen-specific TT-induced responses from spontaneous responses, but that intracytoplasmic cytokine detection by flow cytometry was the most efficient method to detect re-stimulating effects.

Suitable cytokine-inducing agents include, but are not limited to:
- An immunostimulatory oligonucleotide, such as one containing a CpG motif (a dinucleotide sequence containing an unmethylated cytosine linked by a phosphate bond to a guanosine), or a double-stranded RNA, or an oligonucleotide containing a palindromic sequence,_or an oligonucleotide containing a poly(dG) sequence.
- 3-O-deacylated monophosphoryl lipid A ('3dMPL', also known as 'MPL™') [114-117].
- An imidazoquinoline compound, such as Imiquimod ("R-837") [118,119], Resiquimod ("R-848") [120], and their analogs; and salts thereof (*e.g*. the hydrochloride salts). Further details about immunostimulatory imidazoquinolines can be found in references 121 to 125.
- A thiosemicarbazone compound, such as those disclosed in reference 126. Methods of formulating, manufacturing, and screening for active compounds are also described in reference 126. The thiosemicarbazones are particularly effective in the stimulation of human peripheral blood mononuclear cells for the production of cytokines, such as TNF-α.
- A tryptanthrin compound, such as those disclosed in reference 127. Methods of formulating, manufacturing, and screening for active compounds are also described in reference 127. The thiosemicarbazones are particularly effective in the stimulation of human peripheral blood mononuclear cells for the production of cytokines, such as TNF-α.
- A nucleoside analog, such as: (a) Isatorabine (ANA-245; 7-thia-8-oxoguanosine): 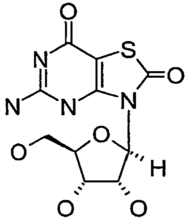 and prodrugs thereof; (b) ANA975; (c) ANA-025-1; (d) ANA380; (e) the compounds disclosed in references 128 to 130; (f) a compound having the formula: 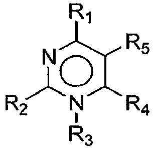 wherein:
   R₁ and R₂ are each independently H, halo, -NRₐR_{b}, -OH, C₁₋₆ alkoxy, substituted C₁₋₆ alkoxy, heterocyclyl, substituted heterocyclyl, C₆₋₁₀ aryl, substituted C₆₋₁₀ aryl, C₁₋₆ alkyl, or substituted C₁₋₆ alkyl;
   R₃ is absent, H, C₁₋₆ alkyl, substituted C₁₋₆ alkyl, C₆₋₁₀ aryl, substituted C₆₋₁₀ aryl, heterocyclyl, or substituted heterocyclyl;
   R₄ and R₅ are each independently H, halo, heterocyclyl, substituted heterocyclyl, -C(O)-R_{d}, C₁₋₆ alkyl, substituted C₁₋₆ alkyl, or bound together to form a 5 membered ring as in R₄₋₅: 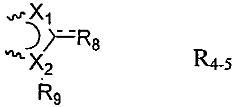 the binding being achieved at the bonds indicated by a 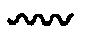
   X₁ and X₂ are each independently N, C, O, or S;
   R₈ is H, halo, -OH, C₁₋₆ alkyl, C₂₋₆ alkenyl, C₂₋₆ alkynyl, -OH, -NRₐR_{b}, -(CH₂)ₙ-O-R_{c}, -O-(C₁₋₆ alkyl), -S(O)ₚRₑ, or -C(O)-R_{d};
   R₉ is H, C₁₋₆ alkyl, substituted C₁₋₆ alkyl, heterocyclyl, substituted heterocyclyl or R₉ₐ, wherein R₉ₐ is: 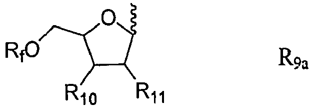 the binding being achieved at the bond indicated by a 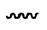
   R₁₀ and R₁₁ are each independently H, halo, C₁₋₆ alkoxy, substituted C₁₋₆ alkoxy, - NRₐR_{b}, or -OH;
   each Rₐ and R_{b} is independently H, C₁₋₆ alkyl, substituted C₁₋₆ alkyl, -C(O)R_{d}, C₆₋₁₀ aryl;
   each R_{c} is independently H, phosphate, diphosphate, triphosphate, C₁₋₆ alkyl, or substituted C₁₋₆ alkyl;
   each R_{d} is independently H, halo, C₁₋₆ alkyl, substituted C₁₋₆ alkyl, C₁₋₆ alkoxy, substituted C₁₋₆ alkoxy, -NH₂, -NH(C₁₋₆ alkyl), -NH(substituted C₁₋₆ alkyl), -N(C₁₋₆ alkyl)₂, -N(substituted C₁₋₆ alkyl)₂, C₆₋₁₀ aryl, or heterocyclyl;
   each Rₑ is independently H, C₁₋₆ alkyl, substituted C₁₋₆ alkyl, C₆₋₁₀ aryl, substituted C₆₋₁₀ aryl, heterocyclyl, or substituted heterocyclyl;
   each R_{f} is independently H, C₁₋₆ alkyl, substituted C₁₋₆ alkyl, -C(O)R_{d}, phosphate, diphosphate, or triphosphate;
   each n is independently 0, 1, 2, or 3;
   each p is independently 0, 1, or 2; or
   or (g) a pharmaceutically acceptable salt of any of (a) to (f), a tautomer of any of (a) to (f), or a pharmaceutically acceptable salt of the tautomer.
- Loxoribine (7-allyl-8-oxoguanosine) [131].
- Compounds disclosed in reference 132, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [133,134], Hydrapthalamide compounds, Benzophenone compounds, Isoxazole compounds, Sterol compounds, Quinazilinone compounds, Pyrrole compounds [135], Anthraquinone compounds, Quinoxaline compounds, Triazine compounds, Pyrazalopyrimidine compounds, and Benzazole compounds [136].
- A polyoxidonium polymer [137,138] or other N-oxidized polyethylene-piperazine derivative.
- Compounds disclosed in reference 139.
- An aminoalkyl glucosaminide phosphate derivative, such as RC-529 [140,141].
- A CD1d ligand, such as an α-glycosylceramide [142-149] (*e.g*. α-galactosylceramide), phytosphingosine-containing α-glycosylceramides, OCH, KRN7000 [(2S,3S,4R)-1-O-(α-D-galactopyranosyl)-2-(N-hexacosanoylamino)-1,3,4-octadecanetriol], CRONY-101, 3"-O-sulfo-galactosylceramide, *etc.*
- A phosphazene, such as poly[di(carboxylatophenoxy)phosphazene] ("PCPP") as described, for example, in references 150 and 151.
- Small molecule immunopotentiators (SMIPs) such as:
   N2-methyl-1-(2-methylpropyl)-1H-imidazo[4,5-c]quinoline-2,4-diamine
   N2,N2-dimethyl-1-(2-methylpropyl)-1H-imidazo[4,5-c]quinoline-2,4-diamine
   N2-ethyl-N2-methyl-1-(2-methylpropyl)-1H-imidazo[4,5-c]quinoline-2,4-diamine
   N2-methyl-1-(2-methylpropyl)-N2-propyl-1H-imidazo[4,5-c]quinoline-2,4-diamine
   1-(2-methylpropyl)-N2-propyl-1H-imidazo[4,5-c]quinoline-2,4-diamine
   N2-butyl-1-(2-methylpropyl)-1H-imidazo[4,5-c]quinoline-2,4-diamine
   N2-butyl-N2-methyl-1-(2-methylpropyl)-1H-imidazo[4,5-c]quinoline-2,4-diamine
   N2-methyl-1-(2-methylpropyl)-N2-pentyl-1H-imidazo[4,5-c]quinoline-2,4-diamine
   N2-methyl-1-(2-methylpropyl)-N2-prop-2-enyl-1H-imidazo[4,5-c]quinoline-2,4-diamine
   1-(2-methylpropyl)-2-[(phenylmethyl)thio]-1H-imidazo[4,5-c]quinolin-4-amine
   1-(2-methylpropyl)-2-(propylthio)-1H-imidazo[4,5-c]quinolin-4-amine
   2-[[4-amino-1-(2-methylpropyl)-1H-imidazo[4,5-c]quinolin-2-yl](methyl)amino]ethanol
   2-[[4-amino-1-(2-methylpropyl)-1H-imidazo[4,5-c]quinolin-2-yl](methyl)amino]ethyl acetate
   4-amino-1-(2-methylpropyl)-1,3-dihydro-2H-imidazo[4,5-c]quinolin-2-one
   N2-butyl-1-(2-methylpropyl)-N4,N4-bis(phenylmethyl)-1H-imidazo[4,5-c]quinoline-2,4-diamine
   N2-butyl-N2-methyl-1-(2-methylpropyl)-N4,N4-bis(phenylmethyl)-1H-imidazo[4,5-c]quinoline-2,4-diamine
   N2-methyl-1-(2-methylpropyl)-N4,N4-bis(phenylmethyl)-1H-imidazo[4,5-c]quinoline-2,4-diamine
   N2,N2-dimethyl-1-(2-methylpropyl)-N4,N4-bis(phenylmethyl)-1H-imidazo[4,5-c]quinoline-2,4-diamine
   -{4-amino-2-[methyl(propyl)amino]-1H-imidazo[4,5-c]quinolin-1-yl}-2-methylpropan2-ol
   1-[4-amino-2-(propylamino)-1H-imidazo[4,5-c]quinolin-1-yl]-2-methylpropan-2-ol
   N4,N4-dibenzyl-1-(2-methoxy-2-methylpropyl)-N2-propyl-1H-imidazo[4,5-c]quinoline-2,4-diamine.

The cytokine-inducing agents for use in the present invention may be modulators and/or agonists of Toll-Like Receptors (TLR). For example, they may be agonists of one or more of the human TLR1, TLR2, TLR3, TLR4, TLR7, TLR8, and/or TLR9 proteins. Preferred agents are agonists of TLR7 (*e.g*. imidazoquinolines) and/or TLR9 (*e.g*. CpG oligonucleotides). These agents are useful for activating innate immunity pathways.

The cytokine-inducing agent can be added to the composition at various stages during its production. For example, it may be within an antigen composition, and this mixture can then be added to an oil-in-water emulsion. As an alternative, it may be within an oil-in-water emulsion, in which case the agent can either be added to the emulsion components before emulsification, or it can be added to the emulsion after emulsification. Similarly, the agent may be coacervated within the emulsion droplets. The location and distribution of the cytokine-inducing agent within the final composition will depend on its hydrophilic/lipophilic properties *e.g*. the agent can be located in the aqueous phase, in the oil phase, and/or at the oil-water interface.

The cytokine-inducing agent can be conjugated to a separate agent, such as an antigen (*e.g*. CRM197). A general review of conjugation techniques for small molecules is provided in ref. 152. As an alternative, the adjuvants may be non-covalently associated with additional agents, such as by way of hydrophobic or ionic interactions.

Two preferred cytokine-inducing agents are (a) immunostimulatory oligonucleotides and (b) 3dMPL.

Immunostimulatory oligonucleotides can include nucleotide modifications/analogs such as phosphorothioate modifications and can be double-stranded or (except for RNA) single-stranded. References 153, 154 and 155 disclose possible analog substitutions *e.g*. replacement of guanosine with 2'-deoxy-7-deazaguanosine. The adjuvant effect of CpG oligonucleotides is further discussed in refs. 156-161. A CpG sequence may be directed to TLR9, such as the motif GTCGTT or TTCGTT [162]. The CpG sequence may be specific for inducing a Th1 immune response, such as a CpG-A ODN (oligodeoxynucleotide), or it may be more specific for inducing a B cell response, such a CpG-B ODN. CpG-A and CpG-B ODNs are discussed in refs. 163-165. Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, references 162 & 166-168. A useful CpG adjuvant is CpG7909, also known as ProMune™ (Coley Pharmaceutical Group, Inc.).

As an alternative, or in addition, to using CpG sequences, TpG sequences can be used [169]. These oligonucleotides may be free from unmethylated CpG motifs.

The immunostimulatory oligonucleotide may be pyrimidine-rich. For example, it may comprise more than one consecutive thymidine nucleotide (*e.g*. TTTT, as disclosed in ref. 169), and/or it may have a nucleotide composition with >25% thymidine (*e.g*. >35%, >40%, >50%, >60%, >80%, *etc*.). For example, it may comprise more than one consecutive cytosine nucleotide (*e.g*. CCCC, as disclosed in ref. 169), and/or it may have a nucleotide composition with >25% cytosine (*e.g*. >35%, >40%, >50%, >60%, >80%, *etc*.). These oligonucleotides may be free from unmethylated CpG motifs.

Immunostimulatory oligonucleotides will typically comprise at least 20 nucleotides. They may comprise fewer than 100 nucleotides.

3dMPL (also known as 3 de-O-acylated monophosphoryl lipid A or 3-O-desacyl-4'-monophosphoryl lipid A) is an adjuvant in which position 3 of the reducing end glucosamine in monophosphoryl lipid A has been de-acylated. 3dMPL has been prepared from a heptoseless mutant of *Salmoella minnesota,* and is chemically similar to lipid A but lacks an acid-labile phosphoryl group and a base-labile acyl group. It activates cells of the monocyte/macrophage lineage and stimulates release of several cytokines, including IL-1, IL-12, TNF-α and GM-CSF (see also ref. 170). Preparation of 3dMPL was originally described in reference 171.

3dMPL can take the form of a mixture of related molecules, varying by their acylation (*e.g*. having 3, 4, 5 or 6 acyl chains, which may be of different lengths). The two glucosamine (also known as 2-deoxy-2-amino-glucose) monosaccharides are N-acylated at their 2-position carbons (*i.e*. at positions 2 and 2'), and there is also O-acylation at the 3' position. The group attached to carbon 2 has formula -NH-CO-CH₂-CR¹R^{1'}. The group attached to carbon 2' has formula -NH-CO-CH₂-CR²R^{2'}. The group attached to carbon 3' has formula -O-CO-CH₂-CR³R^{3'}. A representative structure is:

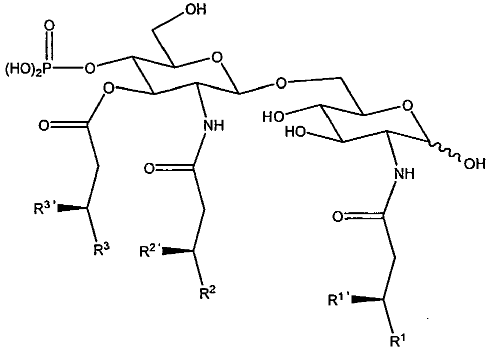

Groups R¹, R² and R³ are each independently -(CH₂)ₙ-CH₃. The value of *n* is preferably between 8 and 16, more preferably between 9 and 12, and is most preferably 10.

Groups R^{1'}, R^{2'} and R^{3'} can each independently be: (a) -H; (b) -OH; or (c) -O-CO-R⁴,where R⁴ is either -H or -(CH₂)ₘ-CH₃, wherein the value of *m* is preferably between 8 and 16, and is more preferably 10, 12 or 14. At the 2 position, *m* is preferably 14. At the 2' position, *m* is preferably 10. At the 3' position, *m* is preferably 12. Groups R^{1'}, R^{2'} and R^{3'} are thus preferably -O-acyl groups from dodecanoic acid, tetradecanoic acid or hexadecanoic acid.

When all of R^{1'}, R^{2'}and R^{3'} are -H then the 3dMPL has only acyl chains (one on each of positions 2, 2' and 3'). When only two of R^{1'}, R^{2'} and R^{3'} are -H then the 3dMPL can have 4 acyl chains. When only one of R^{1'}, R^{2'} and R^{3'} is -H then the 3dMPL can have 5 acyl chains. When none of R^{1'}, R^{2'} and R^{3'} is -H then the 3dMPL can have 6 acyl chains. The 3dMPL adjuvant used according to the invention can be a mixture of these forms, with from 3 to 6 acyl chains, but it is preferred to include 3dMPL with 6 acyl chains in the mixture, and in particular to ensure that the hexaacyl chain form makes up at least 10% by weight of the total 3dMPL *e.g*. ≥20%, ≥30%, ≥40%, ≥50% or more. 3dMPL with 6 acyl chains has been found to be the most adjuvant-active form.

Thus the most preferred form of 3dMPL for inclusion in compositions of the invention is:

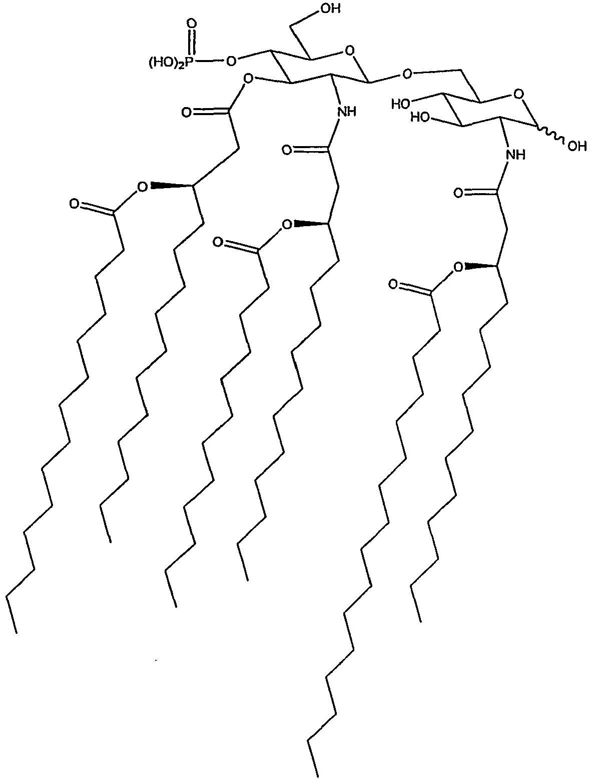

Where 3dMPL is used in the form of a mixture then references to amounts or concentrations of 3dMPL in compositions of the invention refer to the combined 3dMPL species in the mixture.

In aqueous conditions, 3dMPL can form micellar aggregates or particles with different sizes *e.g*. with a diameter <150nm or >500nm. Either or both of these can be used with the invention, and the better particles can be selected by routine assay. Smaller particles (*e.g*. small enough to give a clear aqueous suspension of 3dMPL) are preferred for use according to the invention because of their superior activity [172]. Preferred particles have a mean diameter less than 220nm, more preferably less than 200nm or less than 150nm or less than 120nm, and can even have a mean diameter less than 100 nm. In most cases, however, the mean diameter will not be lower than 50nm. These particles are small enough to be suitable for filter sterilization. Particle diameter can be assessed by the routine technique of dynamic light scattering, which reveals a mean particle diameter. Where a particle is said to have a diameter of x nm, there will generally be a distribution of particles about this mean, but at least 50% by number (*e*.*g*. ≥60%, ≥70%, ≥80%, ≥90%, or more) of the particles will have a diameter within the range *x*±25%.

3dMPL can advantageously be used in combination with an oil-in-water emulsion. Substantially all of the 3dMPL may be located in the aqueous phase of the emulsion.

The 3dMPL can be used on its own, or in combination with one or more further compounds. For example, it is known to use 3dMPL in combination with the QS21 saponin [173] (including in an oil-in-water emulsion [174]), with an immunostimulatory oligonucleotide, with both QS21 and an immunostimulatory oligonucleotide, with aluminum phosphate [175], with aluminum hydroxide [176], or with both aluminum phosphate and aluminum hydroxide.

### Fatty adjuvants

Fatty adjuvants that can be used with the invention include the oil-in-water emulsions described above, and also include, for example:
- A compound of formula I, II or III, or a salt thereof: 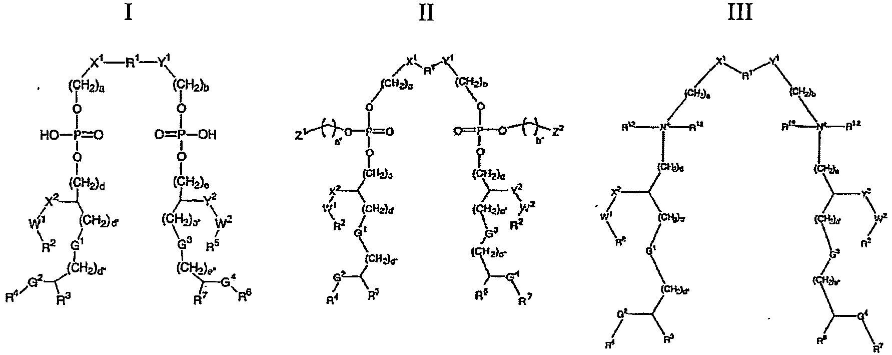 as defined in reference 177, such as 'ER 803058', `ER 803732', 'ER 804053', ER 804058', 'ER 804059', 'ER 804442', 'ER 804680', 'ER 804764', ER 803022 or 'ER 804057' *e.g.*: 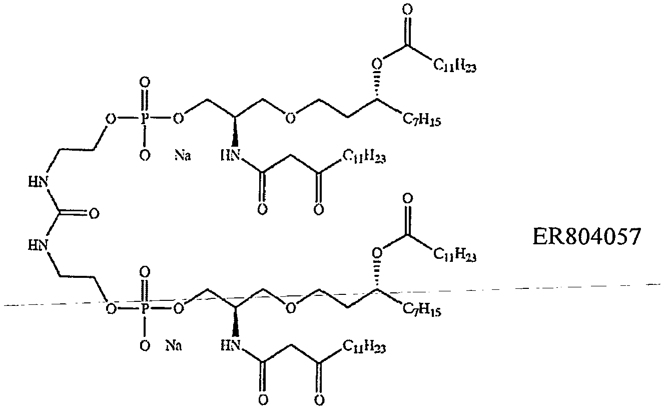 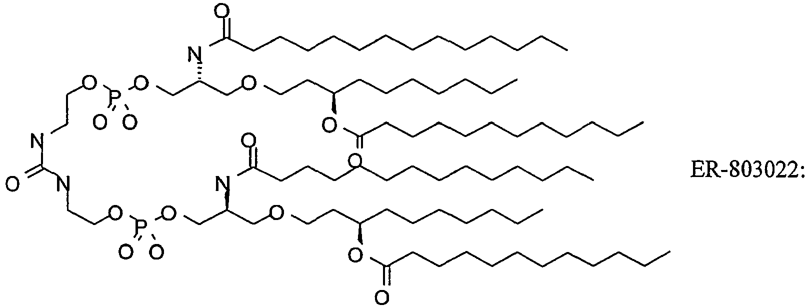
- Derivatives of lipid A from *Escherichia coli* such as OM-174 (described in refs. 178 & 179).
- A formulation of a cationic lipid and a (usually neutral) co-lipid, such as aminopropyl-dimethyl-myristoleyloxy-propanaminium bromide-diphytanoylphosphatidyl-ethanolamine ("Vaxfectin™") or aminopropyl-dimethyl-bis-dodecyloxy-propanaminium bromide-dioleoylphosphatidyl-ethanolamine ("GAP-DLRIE:DOPE"). Formulations containing (±)-N-(3-aminopropyl)-N,N-dimethyl-2,3-bis(syn-9-tetradeceneyloxy)-1-propanaminium salts are preferred [180].
- 3-O-deacylated monophosphoryl lipid A (see above).
- Compounds containing lipids linked to a phosphate-containing acyclic backbone, such as the TLR4 antagonist E5564 [181,182]:

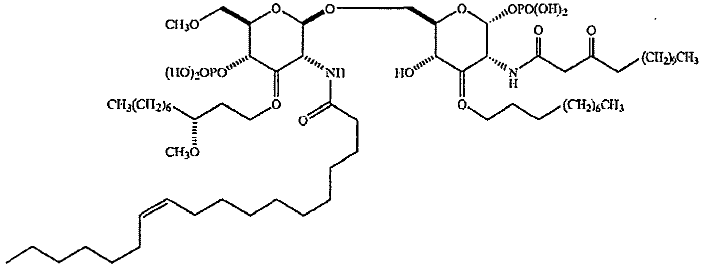

### Aluminum salt adjuvants

The adjuvants known as aluminum hydroxide and aluminum phosphate, may be used. These names are conventional, but are used for convenience only, as neither is a precise description of the actual chemical compound which is present (*e.g*. see chapter 9 of reference 101). The invention can use any of the "hydroxide" or "phosphate," adjuvants that are in general use as adjuvants.

The adjuvants known as "aluminium hydroxide" are typically aluminium oxyhydroxide salts, which are usually at least partially crystalline. Aluminium oxyhydroxide, which can be represented by the formula AlO(OH), can be distinguished from other aluminium compounds, such as aluminium hydroxide Al(OH)₃, by infrared (IR) spectroscopy, in particular by the presence of an adsorption band at 1070cm⁻¹ and a strong shoulder at 3090-3100cm⁻¹ [chapter 9 of ref. 101]. The degree of crystallinity of an aluminium hydroxide adjuvant is reflected by the width of the diffraction band at half height (WHH), with poorly-crystalline particles showing greater line broadening due to smaller crystallite sizes. The surface area increases as WHH increases, and adjuvants with higher WHH values have been seen to have greater capacity for antigen adsorption. A fibrous morphology (*e.g.* as seen in transmission electron micrographs) is typical for aluminium hydroxide adjuvants. The pI of aluminium hydroxide adjuvants is typically about 11 *i.e.* the adjuvant itself has a positive surface charge at physiological pH. Adsorptive capacities of between 1.8-2.6 mg protein per mg Al⁺⁺⁺ at pH 7.4 have been reported for aluminium hydroxide adjuvants.

The adjuvants known as "aluminium phosphate" are typically aluminium hydroxyphosphates, often also containing a small amount of sulfate (*i.e*. aluminium hydroxyphosphate sulfate). They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 1.2. Hydroxyphosphates can be distinguished from strict AlPO₄ by the presence of hydroxyl groups. For example, an IR spectrum band at 3164cm⁻¹ (*e.g.* when heated to 200°C) indicates the presence of structural hydroxyls [ch.9 of ref. 101]

The PO₄/Al³⁺ molar ratio of an aluminium phosphate adjuvant will generally be between 0.3 and 1.2, preferably between 0.8 and 1.2, and more preferably 0.95±0.1. The aluminium phosphate will generally be amorphous, particularly for hydroxyphosphate salts. A typical adjuvant is amorphous aluminium hydroxyphosphate with PO₄/Al molar ratio between 0.84 and 0.92, included at 0.6mg Al³⁺/ml. The aluminium phosphate will generally be particulate (*e.g*. plate-like morphology as seen in transmission electron micrographs). Typical diameters of the particles are in the range 0.5-20µm (*e.g*. about 5-10µm) after any antigen adsorption. Adsorptive capacities of between 0.7-1.5 mg protein per mg Al⁺⁺⁺ at pH 7.4 have been reported for aluminium phosphate adjuvants.

The point of zero charge (PZC) of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 *e.g*. about 5.7.

Suspensions of aluminium salts used to prepare compositions of the invention may contain a buffer (*e.g*. a phosphate or a histidine or a Tris buffer), but this is not always necessary. The suspensions are preferably sterile and pyrogen-free. A suspension may include free aqueous phosphate ions *e.g*. present at a concentration between 1.0 and 20 mM, preferably between 5 and 15 mM, and more preferably about 10 mM. The suspensions may also comprise sodium chloride.

The invention can use a mixture of both an aluminium hydroxide and an aluminium phosphate [62]. In this case there may be more aluminium phosphate than hydroxide *e.g*. a weight ratio of at least 2:1 *e.g.* ≥5:1, ≥6:1, ≥7:1, ≥8:1, ≥9:1, *etc.*

The concentration of Al⁺⁺⁺ in a composition for administration to a patient is preferably less than 10mg/ml *e.g. ≤*5 mg/ml, ≤4 mg/ml, <3 mg/ml, ≤2 mg/ml, <1 mg/ml, *etc.* A preferred range is between 0.3 and 1mg/ml. A maximum of 0.85mg/dose is preferred.

As well as including one or more aluminium salt adjuvants, the adjuvant component may include one or more further adjuvant or immunostimulating agents. Such additional components include, but are not limited to: a 3-O-deacylated monophosphoryl lipid A adjuvant ('3d-MPL'); and/or an oil-in-water emulsion. 3d-MPL has also been referred to as 3 de-O-acylated monophosphoryl lipid A or as 3-O-desacyl-4'-monophosphoryl lipid A. The name indicates that position 3 of the reducing end glucosamine in monophosphoryl lipid A is de-acylated. It has been prepared from a heptoseless mutant of *S.minnesota,* and is chemically similar to lipid A but lacks an acid-labile phosphoryl group and a base-labile acyl group. It activates cells of the monocyte/macrophage lineage and stimulates release of several cytokines, including IL-1, IL-12, TNF-α and GM-CSF. Preparation of 3d-MPL was originally described in reference 171, and the product has been manufactured and sold by Corixa Corporation under the name MPL™. Further details can be found in refs 114 to 117.

### Pharmaceutical compositions

Compositions of the invention are pharmaceutically acceptable. They usually include components in addition to the antigens *e.g*. they typically include one or more pharmaceutical carrier(s) and/or excipient(s). A thorough discussion of such components is available in reference 183.

Compositions will generally be in aqueous form.

The composition includes no mercurial material. It may include a preservative such as 2-phenoxyethanol, but preservative-free vaccines are more preferred.

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Compositions will generally have an osmolality of between 200 mOsm/kg and 400 mOsm/kg, preferably between 240-360 mOsm/kg, and will more preferably fall within the range of 290-310 mOsm/kg. Osmolality has previously been reported not to have an impact on pain caused by vaccination [184], but keeping osmolality in this range is nevertheless preferred.

Compositions may include one or more buffers. Typical buffers include: a phosphate buffer; a Tris buffer; a borate buffer; a succinate buffer; a histidine buffer; or a citrate buffer. Buffers will typically be included in the 5-20mM range. The buffer may be in the emulsion's aqueous phase.

The pH of a composition will generally be between 5.0 and 8.1, and more typically between 6.0 and 8.0 *e.g*. 6.5 and 7.5, or between 7.0 and 7.8. A process of the invention may therefore include a step of adjusting the pH of the bulk vaccine prior to packaging.

The composition is sterile. The composition is preferably gluten free.

The vaccine is free from antibiotics (*e.g*. neomycin, kanamycin, polymyxin B).

The composition may include material for a single immunisation, or may include material for multiple immunisations (*i.e*. a 'multidose' composition). Multidose arrangements usually include a preservative in the vaccine. To avoid this need, a vaccine may be contained in a container having an aseptic adaptor for removal of material.

Influenza vaccines are typically administered in a dosage volume of about 0.5ml, although a half dose (*i.e*. about 0.25ml) may be administered to children, and unit doses will be selected accordingly *e.g*. a unit dose to give a 0.5ml dose for administration to a patient.

### Packaging of compositions or kit components

Processes of the invention can include a step in which vaccine is placed into a container, and in particular into a container for distribution for use by physicians. After packaging into such containers, the container is not refrigerated.

Suitable containers for the vaccines include vials, nasal sprays and disposable syringes, which should be sterile.

Where a composition/component is located in a vial, the vial is preferably made of a glass or plastic material. The vial is preferably sterilized before the composition is added to it. To avoid problems with latex-sensitive patients, vials are preferably sealed with a latex-free stopper, and the absence of latex in all packaging material is preferred. The vial may include a single dose of vaccine, or it may include more than one dose (a 'multidose' vial) *e.g*. 10 doses. Preferred vials are made of colorless glass.

A vial can have a cap (*e.g*. a Luer lock) adapted such that a pre-filled syringe can be inserted into the cap, the contents of the syringe can be expelled into the vial, and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed. A vial may have a cap that permits aseptic removal of its contents, particularly for multidose vials.

Where a composition/component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield. Preferred syringes are those marketed under the trade name "Tip-Lok"™.

Containers may be marked to show a half-dose volume *e.g*. to facilitate delivery to children. For instance, a syringe containing a 0.5ml dose may have a mark showing a 0.25ml volume.

Where a glass container (*e.g*. a syringe or a vial) is used, then it is preferred to use a container made from a borosilicate glass rather than from a soda lime glass.

A composition may be combined (*e.g*. in the same box) with a leaflet including details of the vaccine *e.g*. instructions for administration, details of the antigens within the vaccine, *etc.* The instructions may also contain warnings *e.g.* to keep a solution of adrenaline readily available in case of anaphylactic reaction following vaccination, *etc.*

### Methods of treatment, and administration of the vaccine

Compositions of the invention are suitable for administration to human patients.

The invention also provides a composition of the invention for use as a medicament.

The immune response raised by the methods and uses of the invention will generally include an antibody response, preferably a protective antibody response. Methods for assessing antibody responses, neutralising capability and protection after influenza virus vaccination are well known in the art. Human studies have shown that antibody titers against hemagglutinin of human influenza virus are correlated with protection (a serum sample hemagglutination-inhibition titer of about 30-40 gives around 50% protection from infection by a homologous virus) [185]. Antibody responses are typically measured by hemagglutination inhibition, by microneutralisation, by single radial immunodiffusion (SRID), and/or by single radial hemolysis (SRH). These assay techniques are well known in the art.

Compositions of the invention can be administered in various ways. The most preferred immunisation route is by intramuscular injection (*e.g*. into the arm or leg), but other available routes include subcutaneous injection, intranasal [186-188], oral [189], intradermal [190,191], transcutaneous, transdermal [192], *etc.*

Vaccines prepared according to the invention may be used to treat both children and adults. Influenza vaccines are currently recommended for use in pediatric and adult immunisation, from the age of 6 months. Thus the patient may be less than 1 year old, 1-5 years old, 5-15 years old, 15-55 years old, or at least 55 years old. Preferred patients for receiving the vaccines are the elderly (*e.g*. ≥50 years old, ≥60 years old, and preferably ≥65 years), the young (*e.g*. ≤5 years old), hospitalised patients, healthcare workers, armed service and military personnel, pregnant women, the chronically ill, immunodeficient patients, patients who have taken an antiviral compound (*e.g*. an oseltamivir or zanamivir compound; see below) in the 7 days prior to receiving the vaccine, people with egg allergies and people travelling abroad. The vaccines are not suitable solely for these groups, however, and may be used more generally in a population. For pandemic strains, administration to all age groups is preferred.

Preferred compositions of the invention satisfy 1, 2 or 3 of the CPMP criteria for efficacy. In adults (18-60 years), these criteria are: (1) ≥70% seroprotection; (2) ≥40% seroconversion; and/or (3) a GMT increase of ≥2.5-fold. In elderly (>60 years), these criteria are: (1) ≥60% seroprotection; (2) ≥30% seroconversion; and/or (3) a GMT increase of ≥2-fold. These criteria are based on open label studies with at least 50 patients.

Treatment can be by a single dose schedule or a multiple dose schedule. Multiple doses may be used in a primary immunisation schedule and/or in a booster immunisation schedule. In a multiple dose schedule the various doses may be given by the same or different routes *e.g*. a parenteral prime and mucosal boost, a mucosal prime and parenteral boost, *etc.* Administration of more than one dose (typically two doses) is particularly useful in immunologically naïve patients *e.g*. for people who have never received an influenza vaccine before, or for vaccinating against a new HA subtype (as in a pandemic outbreak). Multiple doses will typically be administered at least 1 week apart (*e.g*. about 2 weeks, about 3 weeks, about 4 weeks, about 6 weeks, about 8 weeks, about 12 weeks, about 16 weeks, *etc*.).

Vaccines produced by the invention may be administered to patients at substantially the same time as (*e.g*. during the same medical consultation or visit to a healthcare professional or vaccination centre) other vaccines *e.g*. at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H.influenzae* type b vaccine, an inactivated poliovirus vaccine, a hepatitis B virus vaccine, a meningococcal conjugate vaccine (such as a tetravalent A C W135 Y vaccine), a respiratory syncytial virus vaccine, a pneumococcal conjugate vaccine, *etc*. Administration at substantially the same time as a pneumococcal vaccine and/or a meningococcal vaccine is particularly useful in elderly patients.

Similarly, vaccines of the invention may be administered to patients at substantially the same time as (*e.g*. during the same medical consultation or visit to a healthcare professional) an antiviral compound, and in particular an antiviral compound active against influenza virus (*e.g*. oseltamivir and/or zanamivir). These antivirals include neuraminidase inhibitors, such as a (3R,4R,5S)-4-acetylamino-5-amino-3(1-ethylpropoxy)-1-cyclohexene-1-carboxylic acid or 5-(acetylamino)-4-[(aminoiminomethyl)-amino]-2,6-anhydro-3,4,5-trideoxy-D-glycero-D-galactonon-2-enonic acid, including esters thereof (*e.g*. the ethyl esters) and salts thereof (*e.g*. the phosphate salts). A preferred antiviral is (3R,4R,5S)-4-acetylamino-5-amino-3(1-ethylpropoxy)-1-cyclohexene-1-carboxylic acid, ethyl ester, phosphate (1:1), also known as oseltamivir phosphate (TAMIFLU™).

### General

The term "comprising" encompasses "including" as well as "consisting" *e.g.* a composition "comprising" X may consist exclusively of X or may include something additional *e.g*. X + Y.

The word "substantially" does not exclude "completely" *e.g*. a composition which is "substantially free" from Y may be completely free from Y. Where necessary, the word "substantially" may be omitted from the definition of the invention.

The term "about" in relation to a numerical value x means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc*.

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

Where a cell substrate is used for reassortment or reverse genetics procedures, it is preferably one that has been approved for use in human vaccine production e.g. as in Ph Eur general chapter 5.2.3.

Identity between polypeptide sequences is preferably determined by the Smith-Waterman homology search algorithm as implemented in the MPSRCH program (Oxford Molecular), using an affine gap search with parameters *gap open penalty=12* and *gap extension penalty=1.*

### MODES FOR CARRYING OUT THE INVENTION

Rather than using eggs, influenza A and B viruses were grown in MDCK cells in a suspension culture, following the teaching of references 27 and 47. The culture did not include any antibiotic.

The final culture medium was clarified to provide virions, which were then subjected to chromatography and ultrafiltration/diafiltration.

Rather than use formaldehyde for inactivation, virions in the resulting material were inactivated using β-propiolactone (final concentration 0.05% v/v; incubated for 16-20 hours at 2-8°C, and then hydrolyzed by incubating at 37°C for 2-2.5 hours), following the teaching of reference 59. CTAB was then used to split the virions, and various further processing steps gave a final monovalent bulk vaccine containing purified surface proteins.

The final bulk material contained no mercurial preservative, no an antibiotic, no formaldehyde, and no egg-derived materials.

Individual doses of vaccine were prepared from the bulk, each containing 15µg of HA from a A/H1N1, A/H3N2 and B strain. This vaccine was administered to patients in a clinical trial, with control patients receiving egg-derived Agrippal™ (which can include formaldehyde and trace amounts of antibiotic). The MDCK-derived vaccine was well tolerated, highly immunogenic (immunologically non-inferior to Agrippal), and met CHMP and CBER criteria for assessment of influenza vaccines. Similar immunogenicity and safety profiles were induced by three different lots of the MDCK-derived vaccine, confirming that the cell culture manufacturing technology is able to generate consistent clinical results.

### REFERENCES

[1] Vaccines. (eds. Plotkin & Orenstein). 4th edition, 2004, ISBN: 0-7216-9688-0.
[2] WO97/14434.
[3] Poole & Mussett (1989) J Biol Stand 17:161-71.
[4] Poole et al. (1997) J. Endotoxin Res 4:221-31
[5] WO02/28422.
[6] WO02/067983.
[7] WO02/074336.
[8] WO02/097072.
[9] WO2005/113756.
[10] WO01/21151.
[11] Huckriede et al. (2003) Methods Enzymol 373:74-91.
[12] World Health Organisation (2005) Emerging Infectious Diseases 11(10):1515-21.
[13] Herlocher et al. (2004) J Infect Dis 190(9):1627-30.
[14] Le et al. (2005) Nature 437(7062):1108.
[15] Hoffmann et al. (2002) Vaccine 20:3165-3170.
[16] Subbarao et al. (2003) Virology 305:192-200.
[17] Liu et al. (2003) Virology 314:580-590.
[18] Ozaki et al. (2004) J. Virol. 78:1851-1857.
[19] Webby et al. (2004) Lancet 363:1099-1103.
[20] WO00/60050.
[21] WO01/04333.
[22] US patent 6649372.
[23] Neumann et al. (2005) Proc Natl Acad Sci USA 102:16825-9.
[24] W02006/067211.
[25] WO01/83794.
[26] Hoffmann et al. (2000) Virology 267(2):310-7.
[27] WO97/37000.
[28] Brands et al. (1999) Dev Biol Stand 98:93-100.
[29] Halperin et al. (2002) Vaccine 20:1240-7.
[30] Tree et al. (2001) Vaccine 19:3444-50.
[31] Kistner et al. (1998) Vaccine 16:960-8.
[32] Kistner et al. (1999) Dev Biol Stand 98:101-110.
[33] Bruhl et al. (2000) Vaccine 19:1149-58.
[34] Pau et al. (2001) Vaccine 19:2716-21.
[35] *http:*//*www.atcc.org*/
[36] *http:*//*locus.umdnj.edu*/
[37] WO03/076601.
[38] WO2005/042728.
[39] WO03/043415.
[40] WO01/85938
[41] WO2006/108846
[42] EP-A-1260581 (WO01/64846).
[43] W02006/071563.
[44] W02005/113758.
[45] W02006/027698.
[46] WO03/023021
[47] WO03/023025
[48] WO97/37001.
[49] Treanor et al. (1996) J Infect Dis 173:1467-70.
[50] Keitel et al. (1996) Clin Diagn Lab Immunol 3:507-10.
[51] Lundblad (2001) Biotechnology and Applied Biochemistry 34:195-197.
[52] Guidance for Industry: Bioanalytical Method Validation. U.S. Department of Health and Human Services Food and Drug Administration Center for Drug Evaluation and Research (CDER) Center for Veterinary Medicine (CVM). May 2001.
[53] Ji et al. (2002) Biotechniques. 32:1162-7.
[54] Briggs (1991) J Parenter Sci Technol. 45:7-12.
[55] Lahijani et al. (1998) Hum Gene Ther. 9:1173-80.
[56] Lokteff et al. (2001) Biologicals. 29:123-32.
[57] EP-B-0870508.
[58] US 5948410.
[59] W02007/052163.
[60] US patent 6,372,223.
[61] WO00/15251.
[62] WO01/22992.
[63] Hehme et al. (2004) Virus Res. 103(1-2):163-71.
[64] US patent 6355271.
[65] WO00/23105.
[66] US 5,057,540.
[67] WO2005/002620.
[68] WO96/33739.
[69] EP-A-0109942.
[70] WO96/11711.
[71] WO00/07621.
[72] WO2004/004762.
[73] Barr et al. (1998) Advanced Drug Delivery Reviews 32:247-271.
[74] Sjolanderet et al. (1998) Advanced Drug Delivery Reviews 32:321-338.
[75] Pizza et al. (2000) Int J Med Microbiol 290:455-461.
[76] WO95/17211.
[77] WO98/42375.
[78] Singh et al] (2001) J Cont Release 70:267-276.
[79] WO99/27960.
[80] US 6,090,406
[81] US 5,916,588
[82] EP-A-0626169.
[83] WO99/52549.
[84] WO01/21207.
[85] WO01/21152.
[86] WO02/072012.
[87] Signorelli & Hadden (2003) Int Immunopharmacol 3(8):1177-86.
[88] W02004/064715.
[89] Cooper (1995) Pharm Biotechnol 6:559-80.
[90] W02005/089837.
[91] US patent 6,692,468.
[92] WO00/07647.
[93] WO99/17820.
[94] US patent 5,971,953.
[95] US patent 4,060,082.
[96] EP-A-0520618.
[97] WO98/01174.
[98] WO90/14837.
[99] Podda & Del Giudice (2003) Expert Rev Vaccines 2:197-203.
[100] Podda (2001) Vaccine 19: 2673-2680.
[101] Vaccine Design: The Subunit and Adjuvant Approach (eds. Powell & Newman) Plenum Press 1995 (ISBN 0-306-44867-X).
[102] Vaccine Adjuvants: Preparation Methods and Research Protocols (Volume 42 of Methods in Molecular Medicine series). ISBN: 1-59259-083-7. Ed. O'Hagan.
[103] Allison & Byars (1992) Res Immunol 143:519-25.
[104] Hariharan et al. (1995) Cancer Res 55:3486-9.
[105] US-2007/014805.
[106] WO95/11700.
[107] US patent 6,080,725.
[108] WO2006/113373.
[109] WO2005/097181.
[110] Han et al. (2005) Impact of Vitamin E on Immune Function and Infectious Diseases in the Aged at Nutrition, Immune functions and Health EuroConference, Paris, 9-10 June 2005.
[111] US- 6630161.
[112] Hayden et al. (1998) J Clin Invest 101(3):643-9.
[113] Tassignon et al. (2205) J Immunol Meth 305:188-98*.*
[114] Myers et al. (1990) pages 145-156 of Cellular and molecular aspects of endotoxin reactions*.*
[115] Ulrich (2000) Chapter 16 (pages 273-282) of reference 102.
[116] Johnson et al. (1999) J Med Chem 42:4640-9.
[117] Baldrick et al. (2002) Regulatory Toxicol Pharmacol 35:398-413.
[118] US 4,680,338.
[119] US 4,988,815.
[120] WO92/15582.
[121] Stanley (2002) Clin Exp Dermatol 27:571-577.
[122] Wu et al. (2004) Antiviral Res. 64(2):79-83.
[123] Vasilakos et al. (2000) Cell Immunol. 204(1):64-74.
[124] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.
[125] Jones (2003) Curr Opin Investig Drugs 4:214-218.
[126] WO2004/060308.
[127] WO2004/064759.
[128] US 6,924,271.
[129] US2005/0070556.
[130] US 5,658,731.
[131] US patent 5,011,828.
[132] W02004/87153.
[133] US 6,605,617.
[134] WO02/18383.
[135] WO2004/018455.
[136] WO03/082272.
[137] Dyakonova et al. (2004) Int Immunopharmacol 4(13):1615-23.
[138] FR-2859633.
[139] WO2006/002422.
[140] Johnson et al. (1999) Bioorg Med Chem Lett 9:2273-2278.
[141] Evans et al. (2003) Expert Rev Vaccines 2:219-229.
[142] De Libero et al, Nature Reviews Immunology, 2005, 5: 485-496
[143] US patent 5,936,076.
[144] Oki et al, J. Clin. Investig., 113: 1631-1640
[145] US2005/0192248
[146] Yang et al, Angew. Chem. Int. Ed., 2004,43: 3818-3822
[147] WO2005/102049
[148] Goff et al, J. Am. Chem., Soc., 2004, 126: 13602-13603
[149] WO03/105769
[150] Andrianov et al. (1998) Biomaterials 19:109-115.
[151] Payne et al. (1998) Adv Drug Delivery Review 31:185-196.
[152] Thompson et al. (2003) Methods in Molecular Medicine 94:255-266.
[153] Kandimalla et al. (2003) Nucleic Acids Research 31:2393-2400.
[154] WO02/26757.
[155] WO99/62923.
[156] Krieg (2003) Nature Medicine 9:831-835.
[157] McCluskie et al. (2002) FEMS Immunology and Medical Microbiology 32:179-185.
[158] WO98/40100.
[159] US patent 6,207,646.
[160] US patent 6,239,116.
[161] US patent 6,429,199.
[162] Kandimalla et al. (2003) Biochemical Society Transactions 31 (part 3):654-658.
[163] Blackwell et al. (2003) J Immunol 170:4061-4068.
[164] Krieg (2002) Trends Immunol 23:64-65.
[165] WO01/95935.
[166] Kandimalla et al. (2003) BBRC 306:948-953.
[167] Bhagat et al. (2003) BBRC 300:853-861.
[168] WO03/035836.
[169] WO01/22972.
[170] Thompson et al. (2005) J Leukoc Biol 78:1273-80.
[171] UK patent application GB-A-2220211.
[172] WO 94/21292.
[173] WO94/00153.
[174] WO95/17210.
[175] WO96/26741.
[176] WO93/19780.
[177] WO03/011223.
[178] Meraldi et al. (2003) Vaccine 21:2485-2491.
[179] Pajak et al. (2003) Vaccine 21:836-842.
[180] US-65 86409.
[181] Wong et al. (2003) J Clin Pharmacol 43(7):735-42.
[182] US2005/0215517.
[183] Gennaro (2000) Remington: The Science and Practice of Pharmacy. 20th edition, ISBN: 0683306472.
[184] Nony et al. (2001) Vaccine 27:3645-51.
[185] Potter & Oxford (1979) Br Med Bull 35: 69-75.
[186] Greenbaum et al. (2004) Vaccine 22:2566-77.
[187] Zurbriggen et al. (2003) Expert Rev Vaccines 2:295-304.
[188] Piascik (2003) J Am Pharm Assoc (Wash DC). 43:728-30.
[189] Mann et al. (2004) Vaccine 22:2425-9.
[190] Halperin et al. (1979) Am J Public Health 69:1247-50.
[191] Herbert et al. (1979) J Infect Dis 140:234-8.
[192] Chen et al. (2003) Vaccine 21:2830-6.

## Claims

1. A sterile vaccine comprising an inactivated influenza virus antigen, wherein the vaccine contains no mercurial preservative, no antibiotic, no formaldehyde and no egg-derived materials.

2. The vaccine of any preceding claim, having less than 0.1 IU/ml of endotoxin.

3. The vaccine of any preceding claim, having less than 10ng of host cell DNA per 15µg of haemagglutinin.

4. The vaccine of any preceding claim, wherein the antigen is a split virus antigen.

5. The vaccine of any one of claims 1-3, wherein the antigen is a purified surface glycoprotein antigen.

6. The vaccine of any one of claims 1-3, wherein the antigen is a virosome.

7. The vaccine of any preceding claim, including antigen from more than one influenza virus strain.

8. The vaccine of any preceding claim further comprising an adjuvant.

9. The vaccine of claim 8 wherein the adjuvant is an oil-in-water emulsion.

10. The vaccine of claim 9, wherein the oil-in-water emulsion comprises squalene.

11. A process for preparing a sterile influenza virus antigen formulation which contains no mercurial preservative, no antibiotic, no formaldehyde and no egg-derived materials, comprising the steps of: (i) growing influenza virus in a cell culture system, in the absence of egg-derived materials and of antibiotics; (ii) inactivating the influenza viruses grown in step (i), in the absence of formaldehyde; and (iii) preparing a vaccine antigen formulation from the inactivated influenza viruses, in the absence of thimerosal.

12. The process of claim 11, wherein the influenza viruses in step (ii) are treated with an alkylating agent.

13. The process of claim 11 or claim 12 wherein the influenza viruses are grown in MDCK cells.

14. The process of claim 13 wherein the MDCK cells are MDCK 33016 deposited as DSM ACC2219.

15. The process of any one of claims 10 to 14 wherein step (i) is performed in serum-free media.

## Patentansprüche

1. Steriler Impfstoff, umfassend ein inaktiviertes Influenzavirus-Antigen, wobei der Impfstoff kein quecksilberhaltiges Konservierungsmittel, kein Antibiotikum, kein Formaldehyd und keine aus Eiern stammende Materialien enthält.

2. Impfstoff nach einem vorhergehenden Anspruch, mit weniger als 0,1 IU/ml Endotoxin.

3. Impfstoff nach einem vorhergehenden Anspruch, mit weniger als 10 ng Wirtszellen-DNA pro 15 µg Hämagglutinin.

4. Impfstoff nach einem vorhergehenden Anspruch, wobei es sich bei dem Antigen um ein Spaltvirus-Antigen handelt.

5. Impfstoff nach einem der Ansprüche 1-3, wobei es sich bei dem Antigen um ein gereinigtes Oberflächenglykoprotein-Antigen handelt.

6. Impfstoff nach einem der Ansprüche 1-3, wobei es sich bei dem Antigen um ein Virosom handelt.

7. Impfstoff nach einem vorhergehenden Anspruch, enthaltend Antigen aus mehr als einem Influenzavirus-Stamm.

8. Impfstoff nach einem vorhergehenden Anspruch, ferner umfassend ein Adjuvans.

9. Impfstoff nach Anspruch 8, wobei es sich bei dem Adjuvans um eine Öl-in-Wasser-Emulsion handelt.

10. Impfstoff nach Anspruch 9, wobei die Öl-in-Wasser-Emulsion Squalen umfasst.

11. Verfahren zur Herstellung einer sterilen Influenzavirus-Antigen-Formulierung, die kein quecksilberhaltiges Konservierungsmittel, kein Antibiotikum, kein Formaldehyd und keine aus Eiern stammende Materialien enthält, mit den folgenden Schritten: (i) Kultivieren von Influenzavirus in einem Zellkultursystem, in Abwesenheit von aus Eiern stammenden Materialien und von Antibiotika; (ii) Inaktivieren der in Schritt (i) kultivierten Influenzaviren, in Abwesenheit von Formaldehyd; und (iii) Herstellen einer Antigen-Impfstoffformulierung aus den inaktivierten Influenzaviren, in Abwesenheit von Thimerosal.

12. Verfahren nach Anspruch 11, wobei die Influenzaviren in Schritt (ii) mit einem Alkylierungsmittel behandelt werden.

13. Verfahren nach Anspruch 11 oder Anspruch 12, wobei die Influenzaviren in MDCK-Zellen kultiviert werden.

14. Verfahren nach Anspruch 13, wobei es sich bei den MDCK-Zellen um MDCK 33016, hinterlegt als DSM ACC2219, handelt.

15. Verfahren nach einem der Ansprüche 10 bis 14, wobei Schritt (i) in serumfreien Medien ausgeführt wird.

## Revendications

1. Vaccin stérile comprenant un antigène du virus de la grippe inactivé, **caractérisé en ce que** le vaccin ne contient pas d'agent conservateur mercuriel, d'antibiotique, de formaldéhyde ni de matières issues d'oeufs.

2. Vaccin selon l'une quelconque des revendications précédentes, ayant moins de 0,1 UI/ml d'endotoxine.

3. Vaccin selon l'une quelconque des revendications précédentes, ayant moins de 10 ng d'ADN de cellule hôte par 15 µg d'hémagglutinine.

4. Vaccin selon l'une quelconque des revendications précédentes, **caractérisé en ce que** l'antigène est un antigène de virus fragmenté.

5. Vaccin selon l'une quelconque des revendications 1 à 3, **caractérisé en ce que** l'antigène est un antigène de glycoprotéine de surface purifié.

6. Vaccin selon l'une quelconque des revendications 1 à 3, **caractérisé en ce que** l'antigène est un virosome.

7. Vaccin selon l'une quelconque des revendications précédentes, comportant un antigène de plus d'une souche de virus de la grippe.

8. Vaccin selon l'une quelconque des revendications précédentes, comprenant en outre un adjuvant.

9. Vaccin selon la revendication 8, **caractérisé en ce que** l'adjuvant est une émulsion huile-dans-l'eau.

10. Vaccin selon la revendication 9, **caractérisé en ce que** l'émulsion huile-dans-l'eau comprend du squalène.

11. Procédé de préparation d'une formulation d'antigène de virus de la grippe stérile qui ne contient pas d'agent conservateur mercuriel, d'antibiotique, de formaldéhyde ni de matières issues d'oeuf, comprenant les étapes consistant à : (i) cultiver le virus de la grippe dans un système de culture de cellules, en l'absence de matières issues d'oeuf et d'antibiotique ; (ii) inactiver les virus de la grippe cultivés à l'étape (i), en l'absence de formaldéhyde ; et (iii) préparer une formulation d'antigène de vaccin à partir des virus de la grippe inactivés, en l'absence de thimérosal.

12. Procédé selon la revendication 11, **caractérisé en ce que** les virus de la grippe à l'étape (ii) sont traités avec un agent alkylant.

13. Procédé selon la revendication 11 ou la revendication 12, **caractérisé en ce que** les virus de la grippe sont cultivés dans des cellules MDCK.

14. Procédé selon la revendication 13, **caractérisé en ce que** les cellules MDCK sont MDCK 33016 déposées en tant que DSM ACC2219.

15. Procédé selon l'une quelconque des revendications 10 à 14, **caractérisé en ce que** l'étape (i) est effectuée dans des milieux sans sérum.
